# EUROPEAN PATENT APPLICATION

(11) **EP 4 309 702 A1**
(43) Date of publication of application: **24.01.2024**
(21) Application number: 22186240.2
(22) Date of filing: 21.07.2022
(51) Int. Cl.: A61M 5/20, A61M 5/315

(54) **INJECTION DEVICE WITH PLAY COMPENSATION**

(71) Applicant: Ypsomed AG, 3401 Burgdorf (CH)
(72) Inventor: Bosshard, Simon Martin, 3324 Hindelbank (CH); Schrul, Christian, 3400 Burgdorf (CH); Zumstein, Dominik, 3014 Bern (CH)
(74) Representative: Meier Obertüfer, Jürg

(57) **Abstract**

The present invention is concerned with an injection device with an elongated housing (200) and integrated electronic circuitry (400). An improved design allows a combination of mechanical play compensation and optical signal transmission through the housing by using translucent or transparent thermoplastic elastomer TPE to realise an elastic coupling (500) in an opening (222) of the housing.

## Description

### FIELD OF THE INVENTION

The present invention relates to injection devices, in particular to injectors with an elongated housing and integrated electronic circuitry. An improved design allows a combination of mechanical play compensation and optical signal transmission by using translucent or transparent thermoplastic elastomer TPE.

### BACKGROUND OF THE INVENTION

A variety of diseases exist that require regular treatment by subcutaneous administration of a medicament, and a number of delivery devices have been developed to support a patient in accurately and controllably delivering an amount of drug in a self-administration process. Drug delivery devices include injection devices that are removed from the site of application after each medication event or drug delivery process, as well as infusion devices with a cannula or needle that remains in the skin of the patient for a prolonged period of time. By way of example, diabetes may be treated by administration of insulin by the patients themselves with the help of multi-variable-dose insulin injection pens or infusion pumps. Alternatively, patch injectors, wearable injectors or wearable pumps are patched or adhered to the skin of the patient.

Common to all devices for subcutaneous drug delivery is a reservoir to store the fluid medicament, and a fluid path to bring the drug out of the device and into the subcutaneous tissue of a patient. In a majority of injecting or infusion devices the reservoir is an elongated cartridge, leading to an even more elongated design of the delivery device, for example hand-held devices with a pen-like shape. A direct consequence of an elongated design is that tolerances of mechanical parts involved add up in an axial direction. To allow manufacturing of the housing, and insertion of the reservoir into the injector, the housing is usually split into two or more axially connected parts. The connection may be fixed and directly between housing components - such as a cap latched on top of a tube for closing the housing - or indirect between housing components and inner parts - such as a number of outer tubes or caps latched to an inner structure. The more complex the mechanical function of the injection device, the more challenging the tolerance issues, the higher the number and complexity of mechanical parts involved and consequently the higher the cost of such a device. The requirement for hand-held operation further adds to mechanical stress to the housing in all possible directions and again increases complexity and cost of design. Mechanical stress, naturally applied to all handheld or mobile devices during handling and all kinds of use, has the effect of tearing components slightly apart. A completely rigid design with all fixed connections tend to be bulky and expensive when it comes to manufacturing; it is common practice especially for lightweight devices to use a housing and basic holding structure made of plastic, snap various components together and let them slide apart in a controlled manner during use. The holding structure, or a train of slidingly connected components, is designed to limit the distance said components are allowed to slide apart. In some cases, a degree of mechanical play is required for proper functionality, for example if a reusable add-on is removably attached to a disposable injector for application. Such a design which includes mechanical play of connections and trains of connected components needs to ensure proper operation in all situations and often requires compensation of play at specific interfaces. Without play compensation, the integration of play-sensitive components such as electromechanical switches or optical components may well become impractical.

The most common way to handle mechanical play is to minimise the number of engaging components involved. Rather complex functions are possible, as disclosed for example in EP3506966 A1 (Sanofi Aventis, pub. 2019). This document discloses an add-on for an injection device, providing supplemental functions such as wireless communication. The interface between add-on and injection device is based on a latch; no play compensation is provided, hence no play-sensitive functions are possible.

With increased complexity of mechanical functions, and with small form factors as required for handheld operation, trying to avoid play compensation is increasingly difficult to achieve. In a handheld injection device, an electromechanical switch often only has a single millimeter of travel. Any mechanical function interacting with an electronic circuitry by means of a switch becomes highly dependent on handling and other external forces, and will not reliably work due to the presence of mechanical play. To overcome this problem, an elastic element is typically introduced into a chain of connected components and pre-tensed to keep the components in an initial state. This allows a design with higher tolerances and more play along mechanical connections, while keeping all components in a well-defined position even for operating an electro-mechanical switch.

EP2729201 A1 (Novo Nordisk, pub) discloses an example of such an injection device, where an add-on is latched onto the main housing. A biasing spring compensates the mechanical play and ensures proper function of an electro-mechanical switch. However, such a biasing spring will add to the size of the device and significantly increase manufacturing cost of the add-on or any injection device following this design.

These examples illustrate that there is clearly a need for a robust but cost-effective approach to design an injection device with or without an add-on that includes the compensation of play and is still suitable for small form factors. This is especially true for injection devices including electronic components and an optical interface.

The term "injection device" or "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

The term "injection device" further refers to any device handled by a user to apply an injection, which explicitly includes a configuration where a supplementary add-on device is fixedly or removably attached to an injector to support or enhance the function of said injector.

The terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal "is meant to refer to the direction or the end of the drug delivery device carrying an injection needle or an injection cannula, whereas the term "proximal" is meant to refer to the opposite direction or end pointing away from the needle or cannula.

### DESCRIPTION OF THE INVENTION

It is an objective of the invention to provide a robust and cost-effective play compensation for an injection device, in particular for an injection device with an optical interface.

This objective is achieved by merging a component with a mechanical play compensation function and a component with an optical function into one single unitary component. In a preferred embodiment, a window or light-guide integrated in one housing component is made of elastic but still transparent or translucent material, and configured to double as a biasing means to compensate mechanical play with respect to an other housing component and bias all mechanical components connecting said two housing components. As mentioned before, one housing component may be part of an add-on removably attached to the other housing component. Injection devices with an elongated housing often have a sequence or train of components arranged in an axial direction relative to a central axis, which makes them particularly susceptible to the effects of mechanical play, and consequently makes them preferred candidates to profit from the present invention. As the invention includes an optical function, an optical component such as a lens, a light sensor or a light emitting diode LED may be involved, typically mounted on a printed circuit board PCB which is part of a train of components connected to the housing and biased to a well-defined operating position by the elastic component providing the play compensation. The optical function involves an optical interface on the injection devices, typically an opening in the housing, a transparent or translucent window, or the outer surface of a light-guide. A preferred embodiment is therefore an injection device comprising an elongate housing with a first housing component and a second housing component defining a main axis, with a signal opening configured to let light pass through the housing, encasing an inner assembly comprising a holding structure and an elastic coupling, whereby the second housing component is slidingly connected to the holding structure, and whereby the inner assembly is configured to limit an axial distance between the second housing component and the holding structure to a maximum distance; and whereby the elastic coupling is compressed to maximise the axial distance, biasing and defining the axial position of all components between the holding structure and the second housing component. The inner assembly further comprises a printed circuit board PCB with an optical component, connected to the holding structure. According to the present invention the elastic coupling is configured to mechanically close the signal opening, while allowing visible light to pass from the exterior of the housing to the optical component or vice versa.

The effect of this improved design of a play compensation for an injection device is that no bulky spring, extra spring support structure and no other extra component is needed to provide play compensation, resulting in a smaller and more cost-effective injection device with significantly reduced manufacturing cost while still enabling play-sensitive functions.

A preferred embodiment of the present invention may further include an electro-mechanical switch mounted on the printed circuit board PCB. As the position of the PCB is well defined, the invention makes it possible to realise a robust design with very small travel of the switch in spite of all mechanical play present.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts an injection device according to the present invention
Fig. 1a depicts an integrated injection device without add-on;
Fig. 1b depicts an injection device comprising a disposable injector and an add-on.
- Fig. 2: depicts a cut across the proximal end of the injection device of Fig. 1a to show a preferred embodiment of the inner assembly:
Fig. 2a the injection device in a state before the housing components are connected;
Fig. 2b the injection device in a state with housing components connected.
- Fig.3a: depicts a perspective view of the transparent elastic coupling inside the second housing component:

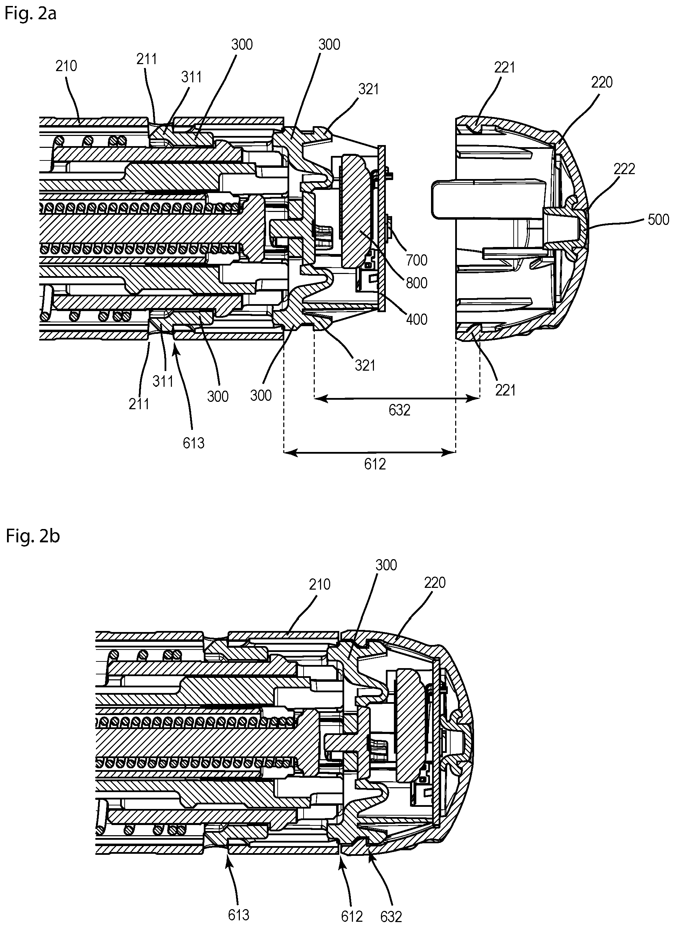

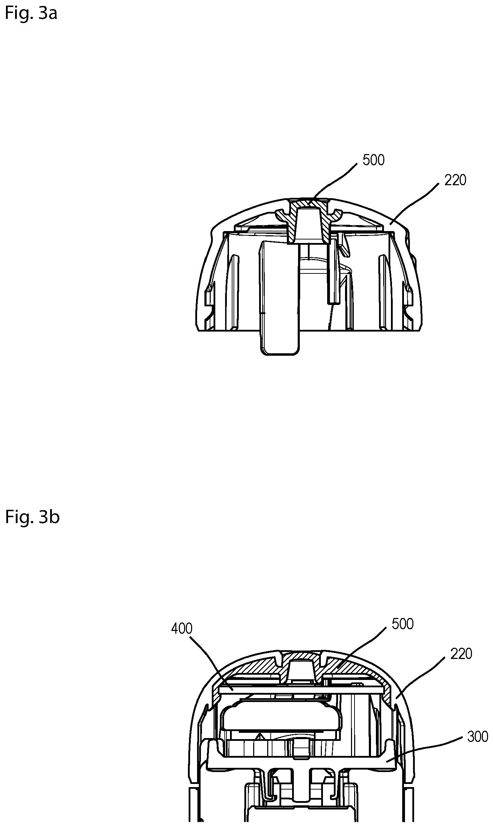

Fig. 3a depicts the second housing component of Fig. 2 with a transparent elastic coupling;
Fig. 3b depicts an alternative embodiment with a second housing component and an alternative transparent elastic coupling.

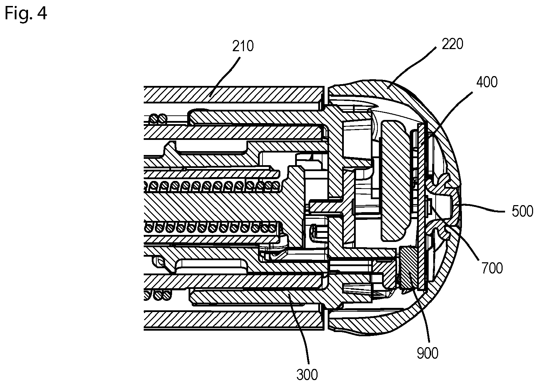

- Fig. 4: depicts the distal end of an injection device with an electro-mechanical switch.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

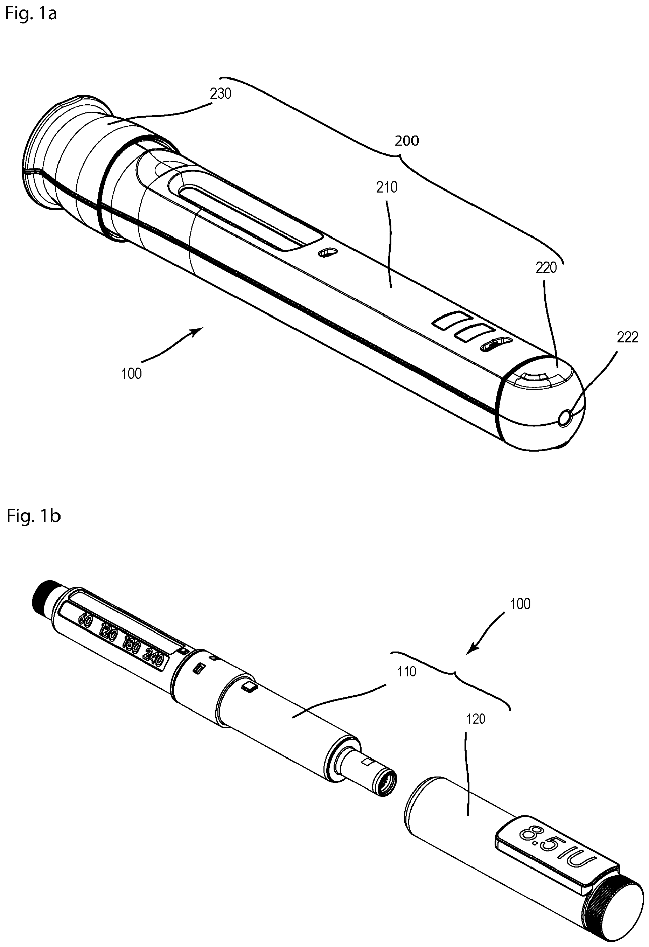

Figure 1a depicts a first preferred embodiment of an injection device (100) according to the present invention. It has an elongated housing (200) encasing an inner assembly configured to realise the injection function. The housing (200) will protect the inner assembly while allowing reliable operation of the injection device. In Fig. 1, the housing (200) comprises a substantially cylindrical first housing component (210), closed on a proximal end by a second housing component (220) and on a distal end by a removable cap (230). Other housing designs with any number of housing components or geometries are suitable for applying the present invention.; the common feature is that there is an axis which can be determined following the elongation of the housing, along which the inner assembly of mechanical components is operatively arranged. Connections between housing components may be fixed or releasable, direct between housing components or indirect via the inner assembly. As connecting components are generally arranged along an axis, play between components will add up and may need compensation for proper and reliable function. The housing may be provided with openings or other features to allow handling and application of the injection device. In a preferred embodiment, where the inner assembly comprises an optical component (700, Fig. 2a), a signal opening (222) is provided in one of the housing components to let light pass through the housing in both directions. A second preferred embodiment is shown in Fig. 1b, where an add-on device (120) is attached to an auto-injector (110) to form an injection device (100). Such an add-on (120) may provide improved functionality or ease of use to the auto-injector (110) when used without the add-on (120). The add-on (120) may be reusable, while the auto-injector (110) may be designed for low cost and single use, forming one injection device (100) when connected together for use. Consequently, the connection between the two devices is typically releasable and will need some play for disconnecting the add-on (120), making this configuration particularly interesting for a play compensation according to the present invention. The housing of the auto-injector (110) will then be the first housing component (210) according to the present invention, and the housing of the add-on (120) the second housing component (220).

The inner assembly inside the housing is configured to provide the functions of an injection device, in particular the injection of a fluid medicament into the body of a patient. For this purpose, the inner assembly typically includes a reservoir, a fluid path, a needle, and an actuator to bring the fluid into the body of a patient. The medicament may be moved out of the reservoir by means of an external energy, e.g. by the user applying a mechanical force to a button, or by an internal energy source driving an actuator, e.g. a pre-loaded spring or a battery providing power to an electric motor. The inner assembly may further include components used for handling the device, such as mechanical interfaces with latches, electromechanical buttons, optical signals or wireless communication. The inner assembly may include an electric power source or battery, and electronic components such as a programmable micro-controller, memory, sensors and control circuitry, typically mounted on a printed circuit board PCB.

An example of an injection device with an elongated housing (200) and an inner assembly is given in EP-3280472-B1 which is incorporated in its entirety into this application. An example of an add-on (120) including electronic components and optical output signals is described in WO2018/085952-A1.

Due to the miniature form factor of electronic components, the interface between electronic and mechanical components is often difficult to realise. An optical signal needs to be aligned with a light-guide, or an electromechanical switch needs to be positioned in all situations and device states with an accuracy matching the travel of the switch. On the other hand, the components of the housing and also operatively coupled components of the inner assembly will allow a certain degree of play, and very often they even need a certain degree for proper and reliable operation. Due to the elongated shape of the injection device, the number of connected components and consequently the amount of play adding up in an axial direction is most pronounced. The mechanical play is a degree of freedom of movement of connected components in an axial direction between a minimum, where a train of slidingly connected components is compressed, and a maximum, where the train of components is stretched to its maximum length. The design of all housing components and components of the inner assembly is made to ensure a controlled sliding movement over a defined axial distance and to provide a defined stop at both ends of the movement. While this play may occur in any direction, the description of the present invention does focus on embodiments with axial play, as they are most relevant for injection devices with an elongated shape.

A typical way to compensate play in a mechanical design is to include at least one elastic component in a critical train of slidingly connected components. The elastic component is pre-compressed during manufacturing of the device and makes sure the axial distance over a train of components is held at a maximum when the device is being used as intended.

Fig. 2a and 2b show a cut across a preferred embodiment of the present invention with a first and a second housing component (210, 220) and an inner assembly comprising various components, in particular a holding structure (300), a PCB (400) with an optical component (700), and a battery (800). In Fig. 2a, the components are shown in a state before final assembly to illustrate the connection of components and trains of components with mechanical play. In this embodiment, the second housing component (220) has fixation means (221) to pair with fixation means (321) of the holding structure (300). The Holding structure (300) further has fixation means (311) to pair with a fixation means (211) of the first housing component (210). Every pair of fixation means is configured to provide a limited minimum and a limited maximum axial distance between the two components connected at one point. In the preferred embodiment of Fig. 2, the fixation means are latches designed into plastic components. However, there may be embodiments with a variety of alternative types of fixation means, such as glue, welding, heat stacking or even releasable fixations like screwing threads or bayonet connections. In a train of connected components, many components may not have fixation means at all: in an elongated housing, they will be stacked together in an axial direction and held in position at minimal distance by the force of the compressed elastic component.

In Fig. 2a and 2b, three trains of slidingly connected components can be identified. A first train of components starts with the holding structure (300) and ends with the second housing component (220). These two components both have fixation means (221, 321) and slidingly connect together, allowing a mechanical play (632). Two components without fixation means are arranged between the holding structure (300) and the second housing component (220): the printed circuit board PCB (400), and the elastic coupling (500). The elastic coupling (500) compensates the play visible as a the gap (632), keeps the axial distance along the first train of components (300, 400, 500, 220) at a maximum and ensures a well defined axial position of all components involved.

A second train of components starts with the first housing component (210) and ends with the holding structure (300). Both components are shown with fixation means (211, 311), no other components are involved in the second train of slidingly connected components. In this embodiment, the axial play at between the first housing component (210) and the holding structure can be held at a level not critical for operation, so the play at the gap (613) remains uncompensated. In a setting with other requirements, like one including water-tight design, a sealing may be used to close the gap (613), which will also provide play compensation for this train of components.

The third train of components can be seen between the first housing component (210) and the second housing component (220). It would comprise a combination of the first and the second train of components and be visible by the gap (612) between the two housing components (210, 220). In this embodiment, no further play compensation is included, but depending on requirements and components involved there are other embodiments, where separate compensation of play over more than one train of components is used. As seen in the example of a preferred embodiment, some of the play in a specific train of components may deliberately remain uncompensated.

As evident from the description of trains of connected mechanical components, variations of the preferred embodiment include injection devices, where the holding structure (300) itself consists of several components, in fact a train of connected components itself. Equally evident are embodiments, where the holding structure (300) is formed together with the first housing component (210) to form one unitary component.

With mechanical play compensated, more critical interfaces can be realised, in particular interfaces with electronic components, such as light emitting diodes LED to send signals out, sensors for visible light to detect optical characteristics of the environment, or electro-mechanical switches to control the operation of the injection device.

The core of the present invention involves combining a mechanical design for play compensation in an injection device with the design of an optical interface. These two functions are jointly realised by providing an elastic coupling which is transparent or translucent to allow visible light pass. While this novel approach may be applied in many different situations, a preferred embodiment is an injection device (100) with a substantially opaque housing (200), a signal opening (222) and an inner assembly as discussed before using Fig. 2. In such an embodiment, the elastic coupling (500) mechanically closes the signal opening (222) and provides play compensation in the first train of slidingly connected components, while allowing visible light to pass from the exterior of the housing (200) to the optical component (7)00 or vice versa. The elastic coupling (500) may also serve as a sealing to protect the inner assembly from the ingress of water or from contamination.

Fig. 3a shows a selection of components of the injection device (100) to illustrate the shape of the elastic coupling (500): the second housing component (220) with the elastic coupling (500) in place cut open and shown in a perspective view. With the elastic coupling (500) made of transparent or translucent material, visible light is free to get in and out of the housing (200). All kind of material may be used for the elastic coupling (500), provided it is elastic and transparent or translucent under the conditions the injection device is being used. A transparent plastic, more specifically a thermoplastic elastomer TPE is a preferred material due to its low cost and ease of manufacturing. Transparent TPE may also be manufactured together with the second housing component (220) using 2K moulding technology, further reducing cost and ease of manufacturing.

With a transparent elastic coupling (500) in place, both play compensation and optical design can be realised with one unitary component. Further functions may be added to the transparent elastic coupling (500), such as sealing any of the gaps (613, 612, 632) to prevent the ingress of water, dust or contaminated air, or flexibly positioning other components of the inner assembly. Fig. 3b shows an embodiment where the transparent elastic component (500) is enlarged sideways to define the radial position of the PCB (400) and provide an improved holding structure for the electronic components in combination with axial play compensation.

Turning to Fig. 4, the embodiment of Fig. 1 is further shown with an electro-mechanical switch (900) mounted on the PCB (400). PCB-mounted switches typically have very small travel, but with the transparent elastic coupling (500) compensating most of the play (612) between the housing components (210, 220), such a switch (900) can safely be operated as part of the inner assembly of the injection device. Standard methods of mechanical design can be applied to define the exact dimensions, shape and elasticity of the coupling element to ensure switching functions as specified for the delivery device. The use of a typical electro-mechanical switch (900) with a switching force of 0.4N and a switching travel of 1mm will for example mean that the elastic coupling (500) is not compressed by more than 1mm when a force of 0.4N is applied to the switch. In an injector according to the present invention, designed using proper skills of the art, manufacturing tolerances or external forces - for example forces occurring during handling or application of the device - will no longer effect the function or accuracy of the device.

Apart from all possible mechanical functions, a transparent elastic coupling (500) may also be configured to provide any other optical function suitable for the chosen material. While shown in Fig. 2 as a window, it may be shape the light in colour or in focus, working as a lens, as a scattering means, as an optical filter or polariser. A special category of embodiments includes a transparent elastic coupling (500) configured to guide visible light from the optical component (700) or vice versa. Solutions with several elastic and non-elastic transparent or translucent components in combination with each other or with other components are also within the scope of the invention as long as they follow the concept of allowing visible light pass through the housing (200) while providing mechanical compensation of play.

By integrating mechanical functions of an elastic coupling with optical functions, all benefits of play compensation can be achieved without an extra component, leading to an improved injection device which can be realised in a smaller form factor with lower manufacturing effort at lower cost, without compromising the accuracy or reliability of drug delivery.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF REFERENCE NUMERALS

- 100: Injection device
- 110: Disposable injector
- 120: Add-on
- 200: Housing
- 210: First housing component
- 211: Fixation (to connecting structure)
- 220: Second housing component
- 221: Fixation (to connecting structure)
- 222: Signal opening
- 230: Removable cap
- 300: Connecting structure
- 311: Fixation (to housing)
- 321: Fixation (to second housing component)
- 400: Printed Circuit Board PCB
- 500: Elastic coupling
- 612: Gap or distance between the first housing component and the second housing component
- 613: Gap or distance between the first housing component and the connecting structure
- 632: Gap or distance between the connecting structure and the second housing component
- 700: Optical component, LED or sensor
- 800: Battery
- 900: Electro-mechanical switch

## Claims

1. An injection device (100) comprising
an elongate housing (200) with a first housing component (210) and a second housing component (220), defining a main axis, with a signal opening (222) configured to let light pass through the housing (200); and
an inner assembly comprising a holding structure (300) and an elastic coupling (500);
whereby the second housing component (220) is slidingly connected to the holding structure (300) in a direction substantially parallel to the main axis, and whereby the inner assembly and/or the second housing component are configured to limit an axial distance (632) between the second housing component (220) and the holding structure (300) to a maximum distance; and whereby
the elastic coupling (500) is compressed to maximise the axial distance (632), defining the axial position of all components between the holding structure (300) and the second housing component (220); and whereby
the inner assembly further comprises a printed circuit board PCB (400) with an optical component (700), arranged between the holding structure (300) and the second housing component (220);
***characterised by*** the elastic coupling (500) being configured to mechanically close the signal opening (222), while allowing visible light to pass from the exterior of the housing (200) to the optical component (700) or vice versa.

2. The injection device according to claim 1, whereby the holding structure (300) comprises a train of connected components.

3. The injection device according to claim 1 or 2, whereby at least a part of the holding structure (300) is formed together with the first housing component (210) to form one unitary component.

4. The injection device according to any preceding claim, whereby the elastic coupling (500) is made of transparent or translucent plastics, preferably a transparent or translucent thermoplastic elastomer TPE.

5. The injection device according to any preceding claim, whereby the optical component (700) is a light emitting diode LED.

6. The injection device according to any preceding claim, whereby the optical component (700) is an optical sensor.

7. The injection device according to claim 5, whereby the PCB (400) further carries an axially operated electromechanical switch (900).

8. The injection device according to any preceding claim, whereby the elastic coupling is manufactured by 2K moulding as a unitary component together with the second housing component (220).

9. The injection device according to any preceding claim, whereby the second housing component (220) is part of an add-on (120) operatively connected to the first housing component (210).

10. The injection device according to any preceding claim, whereby the elastic coupling (500) further functions as a translucent or transparent window.

11. The injection device according to any preceding claim, whereby the elastic coupling (500) further functions as a light-guide.

12. The injection device according to any preceding claim, whereby the elastic coupling (500) further functions as a sealing to prevent the ingress of water, dust or contaminated air into the volume encased by the housing (200).

13. The injection device according to claim 12, whereby the elastic coupling (500) is further configured to close a gap (612) between the first housing component (210) and the second housing component (220) and provide a sealing at the interface between the two housing components.
